# EUROPÄISCHE PATENTSCHRIFT

(11) **EP 0 534 582 B1**
(45) Veröffentlichungstag und Bekanntmachung des Hinweises auf die Patenterteilung: **08.01.1997**
(21) Anmeldenummer: 92250276.0
(22) Anmeldetag: 28.09.1992
(51) Int. Cl.: C07J 1/00, C07C 45/69

(54) **Methylierungs- bzw. Ethylierungsmittel und Verfahren zur 1,4-Addition einer Methyl- bzw. Ethylgruppe an eine Alpha, Beta-ungesättigte Keto-Verbindung**
Methylation or ethylation agent and process for an 1,4-addition of a methyl- or ethylgroup to an alpha, beta-insaturated ketogroup
Agent de méthylation ou de l'éthylation et procédé pour une addition-1,4 d'un groupe méthylique ou éthylique à un groupe cétonique alpha, bêta-insaturé

(30) Priorität: 27.09.1991 DE 4132755
(43) Veröffentlichungstag der Anmeldung: 31.03.1993
(73) Patentinhaber: SCHERING AKTIENGESELLSCHAFT, 13353 Berlin (DE)
(72) Erfinder: Westermann, Jürgen, Dr., W-1000 Berlin 41 (DE); Nickisch, Klaus, Dr., W-1000 Berlin 49 (DE)

(56) Entgegenhaltungen:
- CANADIAN JOURNAL OF CHEMISTRY, Band 45, 1967, Ottawa, M. TANABE et al., Seiten 475-479
- THE JOURNAL OF ORGANIC CHEMISTRY, Band 39, September-Dezember 1974, E.C. ASHBY et al., Seiten 3297-3298
- AUSTRALIAN JOURNAL OF CHEMISTRY, Band 28, 1975, Melbourne, L. BAGNELL et al., Seiten 801-815
- AUSTRALIAN JOURNAL OF CHEMISTRY, Band 28, 1975, Melbourne, L. BAGNELL et al., Seiten 817-820

## Beschreibung

Die vorliegende Erfindung betrifft ein Methylierungs- bzw. Ethylierungsmittel und ein Verfahren zur 1,4-Addition einer Methyl- bzw. Ethylgruppe an ein α,β-ungesättigtes oder ein α,β-doppelt ungesättigtes Keton oder an einen α,β-ungesättigten Aldehyd.

Die 1-Methyleinführung an Steroiden ist ein erster und ein wichtiger Syntheseschritt bei der Herstellung von 1-Methylsteroiden. Beispiele dieser Substanzklasse sind Atamestan (1) (1-Methylandrosta-1,4-dien-3,17-dion), ein Inhibitor der Östrogenbiosynthese (Aromatasehemmer) und Mesterolon (2) (1α-Methylandrosta-17β-ol-3-on), ein Steroid mit androgener Wirkung.

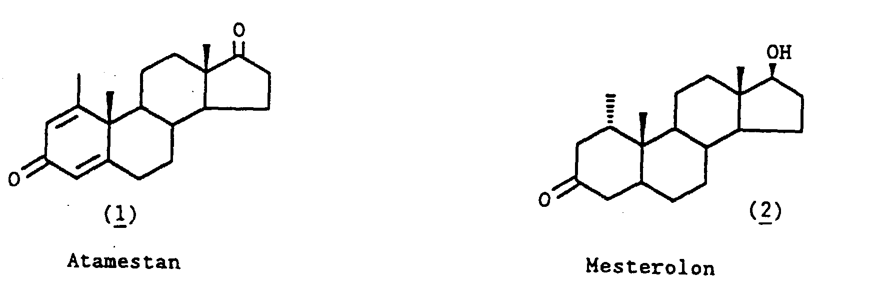

Eine bekannte Methode zur 1-Methyleinführung an z.B. (3) (Androsta-1,4-dien-3,17-dion) zu (4) (1α-Methylandrost-4-en-3,17-dion) ist die Addition von Dimethylkupfer-Lithium, welches aus Methyllithium und Kupfer-I-halogeniden hergestellt wird. Dazu sind molare Mengen des entsprechenden Kupfersalzes erforderlich. Zur Erzielung eines vollständigen Umsatzes bei der Umsetzung zu 4 ist ein deutlicher Überschuß an Reagenz Me₂CuLi erforderlich.

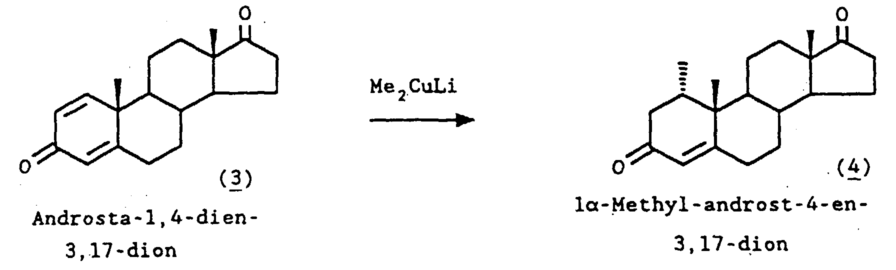

Dieses Verfahren ist Gegenstand der deutschen Patentschriften 204 66 40 und 225 30 87. Ein großes Problem stellen dabei die in molarer Menge anfallenden Kupfer-Salze dar, die aufgearbeitet werden müssen, sich aber durch Filtration nur äußerst schwierig abtrennen lassen.

Der Anfall größerer Mengen an Kupfer-Salzen läßt sich durch Kupfer-I-katalysierte 1,4-Addition mit Methylmagnesiumhalogeniden vermeiden, es findet dabei aber eine unerwünschte 1,2-Addition als Nebenreaktion statt. So läßt sich unter diesen Bedingungen Androsta-1,4-dien-3,17-dion (3) nicht zum gewünschten Produkt 1α-Methyl-androst-4-en-3,17-dion (4) methylieren. Vielmehr bildet sich hier durch Angriff an der 3-Carbonylgruppe und nach Wasserabspaltung aus dem intermediär gebildeten Carbinol 3-Exomethylenandrosta-1,4-dien-17-on (5).

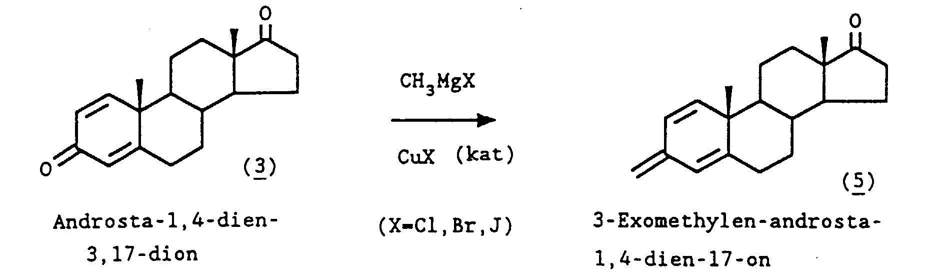

Es gibt in der Literatur nur wenige Beispiele einer 1,4-Addition an ein α,β-doppelt ungesättigtes Carbonylsystem, wie es im Beispiel des Androsta-1,4-dien-3,17-dions (3) der Fall ist. Um direkt aus 3 zu 4 zu gelangen, sind, wie oben dargelegt, stets molare Mengen an Dimethylkupfer-Lithium erforderlich, die aus molaren Mengen Methyllithium und Kupfer-I-halogeniden hergestellt werden müssen.

Als weitere Methode zur Einführung einer Methylgruppe in ein Steroid in 1-Position unter katalytischen Bedingungen ist die im nachfolgenden Schema gezeigte Reaktionsfolge von M. Tanabe und D.F. Crowe in Can. J. Chem. 45, 475 (1967) und in der deutschen Patentschrift 1 223 837 beschrieben.

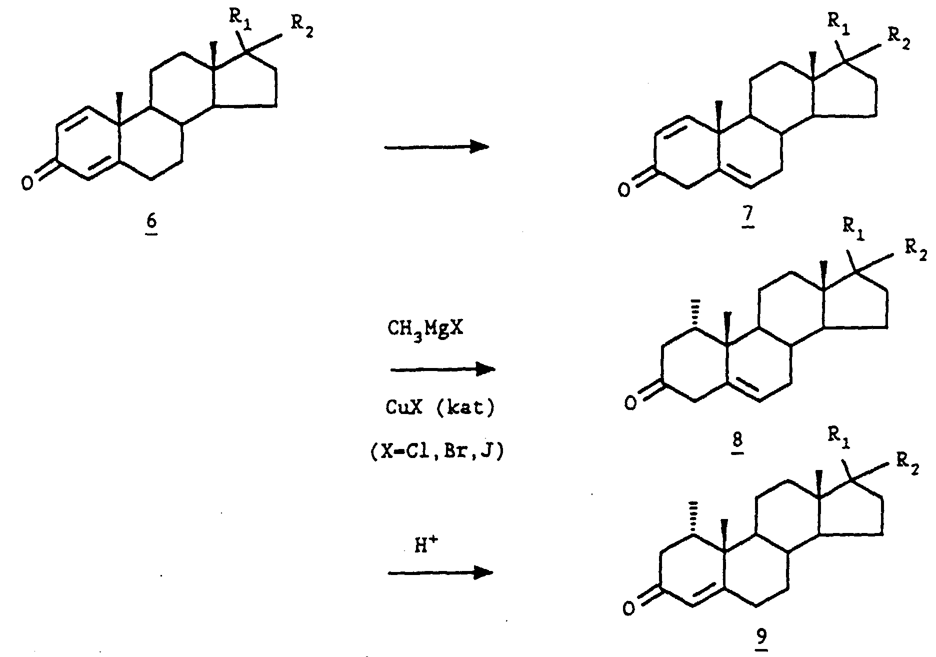

Dazu ist zunächst eine Überführung des 1,4-Dien-Systems 6 in eine 1,5-Dien-Steroidverbindung 7 erforderlich.

Die anschließende Addition an das dekonjugierte 1,5-Dien-System 7 ist unter katalytischen Bedingungen möglich, auch wenn die Ausbeuten bei dem entscheidenden Additionsschritt 7 zu 8 in der Praxis 50% nicht überschreiten. Wegen zusätzlicher Stufenzahl und damit verbundener reduzierter Gesamtausbeute liegt mit der oben beschriebenen mehrstufigen Sequenz insgesamt betrachtet kein vorteilhaftes und ökonomisches Verfahren vor.

Es gibt also bisher für die Einführung einer Methylgruppe in 1-Position in ein 3-Keto-1,4-dien-Steroid (1,4-Addition), beispielsweise Androsta-1,4-dien-3,17-dion (3), kein brauchbares übergangsmetall-katalytisches Verfahren sowie kein geeignetes Methylierungsmittel.

Aufgabe der vorliegenden Erfindung ist es, ein neues Methylierungs- bzw. Ethylierungsmittel zur Verfügung zu stellen sowie ein neues übergangsmetall-katalytisches Verfahren zur 1,4-Addition einer Methyl- bzw. Ethylgruppe an ein α,β-ungesättigtes oder ein α,β-doppelt ungesättigtes Keton oder einen α,β-ungesättigten Aldehyd unter Verwendung dieses neuen Alkylierungsmittels anzugeben. Das zu schaffende Verfahren soll insbesondere zur 1-Methylierung von 3-Keto-1,4-dien-Steroiden geeignet sein.

Diese Aufgabe wird durch das erfindungsgemäße Alkylierungsmittel, welches Trimethylaluminium oder Dimethylzink bzw. Triethylaluminium als Methyl- bzw. Ethylquelle sowie zusätzlich katalytische Mengen einer oder mehrerer Kupfer-I- und/oder Kupfer-II-Verbindungen enthält, gelöst.

Vorzugsweise enthält das Alkylierungsmittel insgesamt 5-10 Mol% der Kupfer-I- und/oder Kupfer-II-Verbindung bezogen auf die zu alkylierende α,β-ungesättigte Ketoverbindung.

Als Kupfer-I- und/oder Kupfer-II-Verbindung(en) kommen in erster Linie eine oder mehrere Verbindung(en) der allgemeinen Formel I

CuX oder CuX₂ (I),

worin X einen einwertigen Rest darstellt und für Chlor, Brom, Iod, Cyano, den Thienyl-, Phenyl-, einen Alkoxy-, Thioalkoxy-, wobei der darin enthaltene Alkylrest 1 bis 8 Kohlenstoffatome besitzt und gegebenenfalls verzweigt und/oder ungesättigt ist, für einen substituierten Alkinylrest R-C≡C-, wobei R einen Phenyl- oder einen gegebenenfalls verzweigten C₁-C₈-Alkylrest bedeutet, oder für den Rest einer anorganischen Säure oder einer Carbonsäure oder für einen zweizähnigen, über Sauerstoff- und/oder Stickstoffatome koordinierenden Komplexliganden, ausgenommen den Liganden Acetylacetonat im Fall des zweiwertigen Kupfers, steht und/oder eine oder mehrere Verbindung(en) der allgemeinen Formel II

Cu₂Y oder CuY (II),

worin Y einen zweiwertigen Rest darstellt und für Sauerstoff oder Schwefel, steht, infrage. Ist X der Rest einer anorganischen Säure, ist beispielsweise an den Hydrogencarbonat-, Hydrogensulfatrest oder an ähnliche Reste gedacht. Als Rest einer organischen Säure kommt vor allem der Acetat-Rest infrage.

Insbesondere ist an Kupfer-I- und/oder II-chlorid oder -bromid sowie Kupfer-I-cyanid als Übergangsmetall-Katalysator gedacht.

Das erfindungsgemäße Alkylierungsmittel kann außerdem zusätzlich bis zu 1 Mol% an Nickel-Salz(en) enthalten; dadurch kann unter bestimmten Bedingungen eine Beschleunigung der Methylierung erreicht werden. Beispielsweise kann Nickel-II-acetylacetonat, Nickel-bis-triphenylphosphan-dichlorid, Nickeldichlorid oder eine ähnliche Verbindung als Nickel-Salz Verwendung finden.

Aluminiumtrimethyl- und -ethyl sowie Zinkdimethyl können als Toluol- oder Hexan-Lösung eingesetzt werden. Wegen der problematischen Handhabung und der Selbstentzündlichkeit der Metallalkyle ist deren Verwendung in Lösung gegenüber der Verwendung in reiner Form vorzuziehen.

Aluminiumorganische Verbindungen gehen unter normalen Rektionsbedingungen ohne Zusatz eines Katalysators nur in geringem Umfang eine 1.2-Addition ein. Es ist literaturbekannt, daß erst unter drastischen Reaktionsbedingungen (höhere Temperaturen) eine solche Reaktion stattfindet. Unter normalen Bedingungen läuft eine solche Reaktion nur ab, wenn ein zweites Molekül Trimethylaluminium zur Verfügung steht. Das erste Molekül Trimethylaluminium komplexiert die Carbonylgruppe, an die so aktivierte Carbonylgruppe addiert dann das zweite Molekül (E.C. Ashby et al., J. Am.Chem. Soc., 90 (1968) 5179). Eine Übersicht über Aluminiumalkyle findet man bei T. Mole und E.A. Jeffry in "Organoaluminium Compounds", Elsevier 1972, Seite 294 ff.
1,4-Addition sind mit Me₂AlJ möglich, verlaufen aber in Konkurrenz zur 1,2-Addition und ohne Zusatz von Kupfer als Katalysator ab (J. Ashley et al., J. Org. Chem., 44 (1979) und sind nicht selektiv.

Bei dem erfindungsgemäßen Verfahren zur 1,4-Addition einer Methyl- bzw. Ethylgruppe an eine α,β-ungesättigte Ketoverbindung wird diese α,β-ungesättigte Ketoverbindung mit Aluminiumtrimethyl oder Zinkdimethyl bzw. Aluminiumtriethyl in Gegenwart einer katalytischen Menge einer oder mehrerer Kupfer-I- und/oder Kupfer-II-Verbindungen alkyliert. Die gewünschte 1,4-Addition läuft glatt ab. Weitere Ausgestaltungen des erfindungsgemäßen Verfahrens ergeben sich aus den Merkmalen der Unteransprüche 10-21.

Für eine solche katalytische Verfahrensweise gibt es in der Literatur noch keine Beispiele.

Der in Aust.J.Chem., 1975,28, S. 801-815 beschriebene Versuch der Methylierung von Isophoron (3,5,5-Trimethyl-2-cyclohexen-1-on) mit Trimethylaluminium in Gegenwart von 6,6 Mol-% Kupfer(II)acetylacetonat (bezogen auf Isophoron) verlief erfolglos. Daß Kupfer(II)-acetylactonat als Methylierungs-Katalysator ungeeignet ist, wird durch die in den Beispielen 18 und 19 beschriebenen Umsetzungsversuche bestätigt.

Beim erfindungsgemäßen Verfahren wird eine Kupfer-I-Verbindung in eine Methyl-Kupfer bzw. Dimethylkupferverbindung überführt, die als solche die 1,4-Addition bewirkt. Nach Übertragung einer Methylgruppe auf das Substrat (Enon) kann das reaktive und 1,4-selektive Kupferreagenz in einem Kreisprozeß aus Trimethylaluminium erneut gebildet werden.

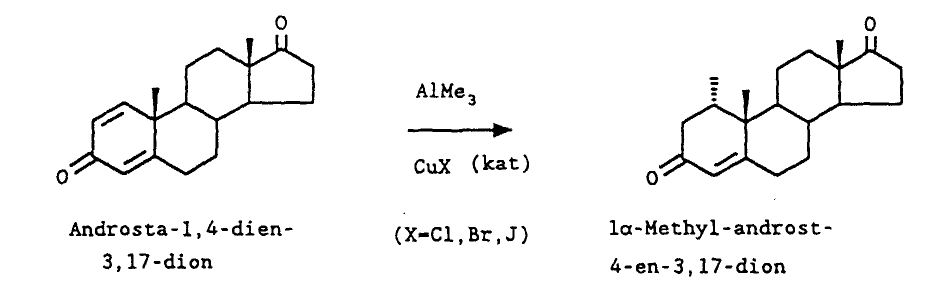

Als Kupfer-I-halogenid wird vorzugsweise das Chlorid oder Bromid eingesetzt. Die Reaktion wird vorzugsweise in Tetrahydrofuran, Dioxan, Dimethoxyethan, Toluol oder auch in Ethylacetat als Lösungsmittel durchgeführt. Überraschend dabei ist, daß unter den gefundenen Reaktionsbedingungen keine Reaktion mit der Carbonsäureestergruppe des Ethylacetates stattfindet. Ein Vorteil von Ethylacetat als Lösungsmittel liegt in der Umweltverträglichkeit dieses Solvens, das aus den natürlich vorkommenden Gruppen Essigsäure und Ethanol zusammengesetzt ist und in der Umwelt in diese natürlichen Moleküle hydrolysiert bzw. abgebaut werden kann.
Neben Kupfer-I-halogeniden sind auch Kupfer-II-halogenide, Kupfer-II-Verbindungen wie CuO und CuS für die Reaktion geeignet. Als gut geeignet haben sich auch Kupfer-II-Komplexe erwiesen, in denen das Kupfer mit Liganden koordiniert ist.
Solche Komplexe der Formel 10 und 11 sind in der Literatur von L. Sacconi et al. in J. Chem. Soc., 1964, 276 beschrieben, die sich vom Salicylaldehyd ableiten und aus diesem herstellen lassen.

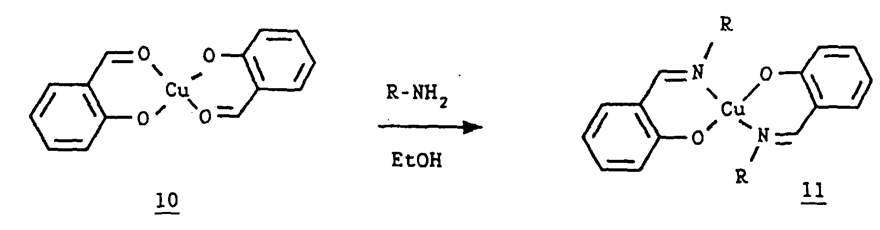

Zugabe von Salicylaldehyd zu einer Kupfer-II-Salz-Lösung liefert nach Filtration den Komplex 10, der nach Isolierung und Umsetzung mit einem Amin wie z.B. Isopropylamin die Schiff'sche Base im Komplex 11 liefert. Analog lassen sich weitere Komplexe herstellen. Bei der Verwendung von Kupfer-II-Verbindungen als Katalysatoren stellen wahrscheinlich auch - durch Reduktion entstandene - Kupfer-I-Verbindungen die aktiven Spezies dar.

Bei all diesen Kupfer-I- und Kupfer-II-Verbindungen ist die Verwendung von katalytischen Anteilen ausreichend. Dabei wird vorzugsweise eine Menge von 5-10 Mol% Kupferverbindung bezogen auf das eingesetzte Enon verwendet.
Erwähnenswert neben der ausgeprägten 1,4-Selektivität bei der Addition an ein 1,4-Dien-3-Ketosteroid ist die hohe 1-Selektivität dieser Addition. Es erfolgt bevorzugt eine Addition in 1-Position des Steroids zu 4, die deutlich gegenüber der sterisch stärker abgeschirmten 5-Position bevorzugt wird.
Das neue Verfahren ist hoch stereoselektiv; der Anteil der als Nebenprodukt gebildeten 5β-Methylverbindung (im Fall der Methylierung von (3) 5β-Methyl-androst-1-en-3,17-dion) liegt unter 5% im Rohprodukt.
Ein weiterer Vorteil des beschriebenen Verfahrens ist, daß Acetylschutzgruppen unter den Reaktionsbedingungen erhalten bleiben. Wie in Beispiel 6 beschrieben, gelingt beispielsweise die Überführung von 17β-Acetoxy-androst-1-en-3-on in 17β-Acetoxy-1α-methyl-5α-androstan-3-on mit einer Ausbeute von 89 % bzw. 95%..
Die Isolierung der Produkte erfolgt vorzugsweise durch Kristallisation der Reaktionsprodukte oder durch Chromatographie. Die Ausbeuten an Produkt betragen bis zu 99% der Theorie.

Die nach dem beschriebenen katalytischen Verfahen aus ADD (3) zugängliche Substanz 1α-Methylandrost-4-en-3,17-dion (4) ist eine wichtige Zwischenstufe zur Synthese von Mesterolon (2).

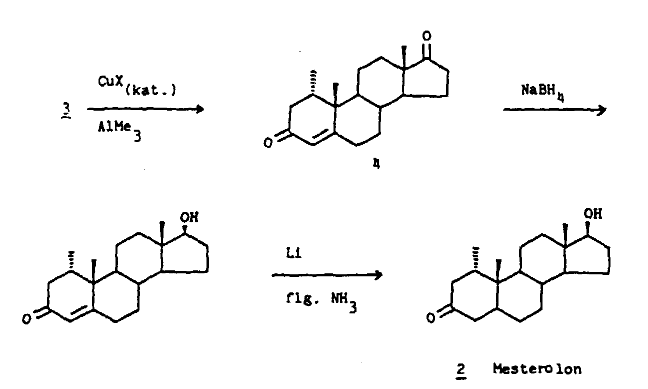

Literatur: DE-1 152 100 B; DE-2 046 640 B; NaBH₄-Reduktion: Fried & Edwards, Organic Reactions in Steroid Chemistry, Vol. I, 1972, S. 61 ff, Van Nostrand Reinhold Company, New York; Birch-Reduktion: Fried & Edwards, Vol.I, S. 39.
Auch 17β-Acetoxy-1α-methyl-5α-androstan-3-on (Beispiel 6) ist als Ausgangsprodukt zur Herstellung von Mesterolon geeignet, welches sich aus ersterem bequem durch Verseifung der 17β-Acylgruppe erhalten läßt.
Wird ein Carbonsäureanhydrid oder -chlorid vor der Aufarbeitung der Reaktion 3 -> 4 zu der Reaktionslösung gegeben, so kann das in der Reaktion vorliegende Enolat als Enolester z.B. 13 abgefangen werden.

Als Carbonsäureanhydrid oder -chlorid kommen die Anhydride der gerad- oder verzweigtkettigen Alkancarbonsäuren mit 2 bis 8 Kohlenstoffatomen, insbesondere der Essigsäure, sowie der Benzoesäure infrage. 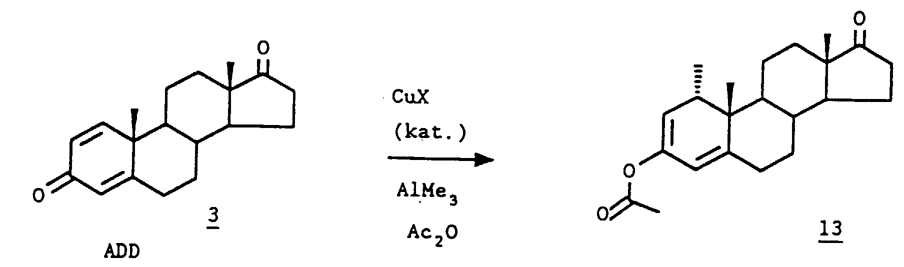 3-Acetoxy-1α-methyl-androsta-2,4-dien-17-on (13) ist ein wichtiges Zwischenprodukt für die Synthese von Atamestan, welches daraus durch stereoselektive 2β-Iodierung sowie nachfolgende Iodwasserstoffabspaltung in hoher Ausbeute erhalten wird (Deutsche Patentanmeldung P 40 15 247.2 und DE-A-37 15 869.4). Diese Darstellung von Atamestan geht aus Beispiel 20 hervor.

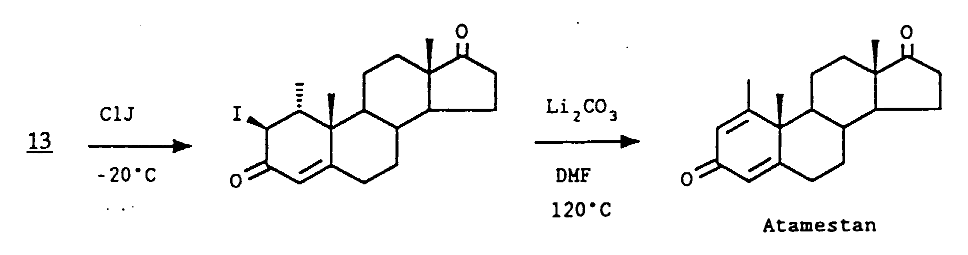

Das erfindungsgemäße Verfahren ist auch geeignet, eine 1,4-Addition an ein einfach ungesättigtes Carbonylsystem wie z.B. 14 einzugehen.

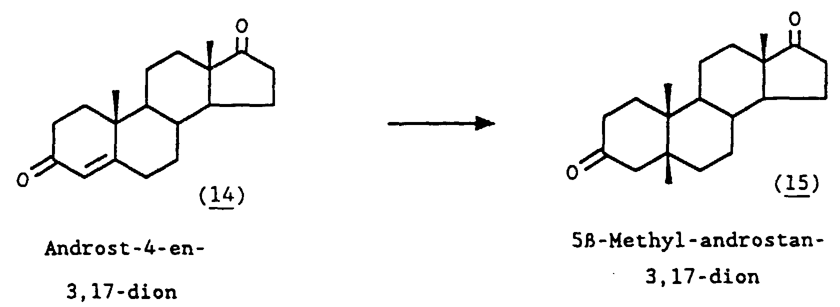

Aus Androst-4-en-3,17-dion (14) entsteht hierbei 5β-Methyl-androstan-3,17-dion (15).

Über die oben aufgezeigte 1,4-Addition an α,β-ungesättigte Keto-Steroide hinaus ist das erfindungsgemäße Verfahren ganz allgemein zur 1,4-Addition einer Methyl- bzw. Ethylgruppe an ein α,β-ungesättigtes Keton anwendbar. Ein Beispiel dazu ist die Umsetzung von Cyclohex-2-en-1-on (16) zu 3-Methylhexanon (17).

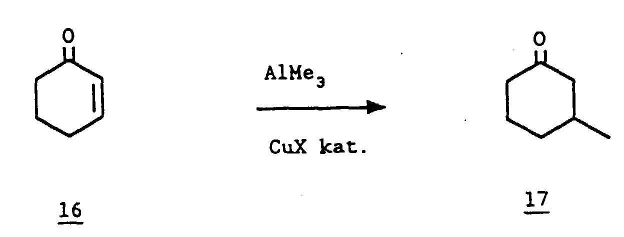

Alle Reaktionen werden vorzugsweise zwischen 0^{o} C und 50^{o} C durchgeführt. Zur Reaktion wird das Keton bzw. Ketosteroid in einem geeigneten Lösungsmittel unter Zusatz von 5-10 Mol% Kupfer-Katalysator unter einer Atmosphäre von Inertgas wie z.B. Stickstoff vorgelegt und Aluminiumtrimethyl (Zinkdimethyl, Aluminiumtriethyl) zwischen 0^{o} C und Raumtemperatur zugegeben. Die Reaktion wird nach ca. 30 - 120 Minuten unter Zusatz von Wasser oder einem niederen Alkohol hydrolysiert und das Produkt anschließend isoliert.

Das erfindungsgemäße Mittel und das beschriebene Verfahren sind nicht auf den Einsatz von Trimethylaluminium oder Dimethylzink bzw. Triethylaluminium als Methyl- bzw. Ethylquelle beschränkt. Es wurde gefunden, daß ganz analog wie vorstehend beschrieben anstelle der genannten Alkylierungs-Reagenzien auch ein Aluminium-Reagenz der Formel Alk₃₋ₙ-AlOEtₙ, worin Alk eine Methyl- oder Ethylgruppe und OEt eine Ethoxygruppe bedeuten und n gleich 1 oder 2 ist, als das die Methyl- bzw. Ethylgruppe liefernde Reagenz innerhalb des erfindungsgemäßen Mittels oder Verfahrens verwendet werden kann. Wenn Alk für eine Methylgruppe steht, so ist n vorzugsweise 1 (Dimethylaluminium-ethoxid).

Nachstehend beschriebene Beispiele sollen die Breite des erfindungsgemäßen Verfahrens aufzeigen. Die dabei erzielten Ausbeuten sollen den Vorteil des neuen katalytischen Verfahrens zur 1-Methyl- bzw. Ethyleinführung an Steroide aufzeigen und die generelle Möglichkeit zur 1,4-Addition an Enonsysteme und Dienonsysteme demonstrieren.

### Beispiel 1: 1α-Methylandrost-4-en-3,17-dion

14.2 g (50 mmol) Androsta-1,4-dien-3,17-dion werden unter Stickstoffatmosphäre in 100 ml wasserfreiem Dioxan gelöst. 716 mg (5 mmol) Kupfer-I-bromid werden zugegeben und die Lösung auf 25 °C erwärmt. Anschließend werden 47 ml (55 mmol) einer 10% igen Lösung Trimethylaluminium in Toluol zu der Reaktion gegeben, so daß die Temperatur nicht über 35 °C ansteigt. Anschließend wird noch 1.5 h bei 35°C nachgerührt. Zur Hydrolyse werden 2.5 ml Wasser gemischt mit 10 ml Dioxan zur Reaktion gegeben und die Lösung noch 15 min nachgerührt. Der anorganische Feststoff wird abgesaugt und mit 30 ml Dioxan nachgewaschen. Nach Einengen der Dioxanlösung werden 17 g Rohprodukt erhalten, die an Kieselgel mit Hexan/Ethylacetat- Gemischen als Eluens chromatographiert werden. Nach Einengen der Fraktionen und Umkristallisation aus Diisopropylether werden 11.66 g 1α-Methylandrost-4-en-3,17-dion (77 % der Theorie) vom Schmelzpunkt 154 °C erhalten.

### Beispiel 2: 5β-Methyl-19-norandrostan-3,17-dion

2.72 g (10 mmol) 19-Norandrost-4-en-3,17-dion werden in 20 ml Dioxan bei 20°C gelöst und mit 143.3 g (1 mmol) Kupfer-I-bromid versetzt. Bei 30°C werden unter Stickstoffatmosphäre 9.4 ml (11 mmol) einer 10%igen Lösung Trimethylaluminium in Toluol zu der Reaktion gegeben und 30 Minuten bei 30 - 40°C gerührt. Zur Hydrolyse werden 0.54 ml Wasser gelöst in 5 ml Dioxan zu der Reaktion gegeben. Es wird 10 Minuten nachgerührt und der Niederschlag abfiltriert. Der Niederschlag wird mit wenig Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 2.6 g 5β-Methyl-19-norandrostan-3,17-dion (90 % der Theorie) vom Schmelzpunkt 155.9 °C erhalten.

### Beispiel 3: 1α-Methyl-17β-acetoxy-androst-4-en-3-on

3.28 g (10 mmol) 17β-Acetoxy-androsta-1,4-dien-3-on werden in 20 ml getrocknetem Dioxan gelöst und mit 143.3 mg (1 mmol) Kupfer-I-bromid versetzt. Unter Stickstoffatmosphäre werden 9.4 ml einer 10%igen Lösung Trimethylaluminium in Toluol über 10 Minuten zu der Reaktion gegeben, daß die Temperatur nicht über 40°C ansteigt. Anschließend wird noch 2 Stunden bei 40°C nachgerührt. Zur Hydrolyse werden 0.54 ml Wasser gelöst in 5 ml Dioxan zur Reaktion gegeben und 10 Minuten nachgerührt. Der Feststoff wird über Kieselgur abgesaugt und mit 20 ml Dioxan nachgewaschen. Das Filtrat wird eingeengt und das Rohprodukt (3.6 g) an Kieselgel mit Hexan/Ethylacetatgemischen als Eluens chromatographiert. Nach Einengen der Fraktionen werden 2.6 g 1α-Methyl-17β-acetoxy-androst-4-en-3-on (75.58 % der Theorie) vom Schmelzpunkt 142° C erhalten.

### Beipiel 4: 5β-Methylandrostan-3,17-dion

2.86 g (10 mmol) Androst-4-en-3,17-dion und 143.3 mg (1 mmol) Kupfer-I-bromid werden in 20 ml trockenem Dioxan gelöst. Bei Raumtemperatur werden zu der Lösung unter Stickstoffatmosphäre 9.4 ml (11 mmol) einer 10%igen Lösung Trimethylaluminium in Toluol über 10 Minuten zu der Reaktion gegeben, so daß die Temperatur nicht über 30°C ansteigt. Anschließend wird noch 2 Stunden bei 30°C nachgerührt. Zur Hydrolyse werden 0.54 ml Wasser gelöst in 5 ml Dioxan zur Reaktion gegeben und 10 Minuten nachgerührt. Der Feststoff wird über Kieselgur abgesaugt und mit 20 ml Dioxan nachgewaschen. Das Filtrat wird eingeengt und das Rohprodukt (3.0 g) an Kieselgel mit Hexan/Ethylacetat als Eluens und steigendem Anteil Ethylacetat chromatographiert. Nach Einengen der Fraktionen werden 2.3 g 5β-Methyl-androsta-3,17-dion (76 % der Theorie vom Schmelzpunkt 135° C erhalten.

### Beispiel 5: 17β-Hydroxy-1α-methyl-androst-4-en-3-on (1α-Methyltestosteron)

2.84 g (10 mmol) Androsta-1,4-dien-3,17-dion werden unter Stickstoffatmosphäre in 20 ml wasserfreiem Dioxan gelöst. 143.3 mg (1 mmol) Kupfer-I-bromid werden zugegeben und die Lösung auf 25 °C erwärmt. Anschließend werden 9.4 ml (11 mmol) einer 10% igen Lösung Trimethylaluminium in Toluol zu der Reaktion gegeben, so daß die Temperatur nicht über 35 °C ansteigt. Anschließend wird noch 1.5 h bei 35°C nachgerührt. Zur Hydrolyse werden 0.54 ml Wasser gemischt mit 5 ml Dioxan vorsichtig zur Reaktion gegeben und die Lösung noch 15 min nachgerührt. Der anorganische Feststoff wird abgesaugt und mit 30 ml Dioxan nachgewaschen. Nach Einengen der Lösung werden 3.1 g 1α-Methylandrost-4-en-3,17-dion erhalten, die in 8 ml Methanol gelöst und auf -5°C Innentemperatur abgekühlt werden. 0.18 g Natriumborhydrid werden zu 0.5 ml auf 5°C gekühltes Wasser gegeben und die Natriumborhydrid-Wasserlösung so zu der Lösung 1α-Methyl-androst-4-en-3,17-dion gegeben, so daß die Temperatur nicht über -5°C ansteigt. Anschließend wird noch 40 Minuten bei -5°C nachgerührt. Nach DC-Kontrolle auf erfolgte Umsetzung werden 0.33 ml Essigsäure in 1 ml Wasser vorsichtig zugegeben bis keine Gasentwicklung mehr feststellbar ist. Die Lösung wird in 50 ml Ethylacetat aufgenommen und 2 mal mit je 20 ml Wasser gewaschen. Die organische Phase wird mit Natriumsulfat getrocknet. Nach Einengen der Lösung und Umkristallisation aus Aceton werden 2.29 g 17β-Hydroxy-1α-methyl-androst-4-en-3-on (75% d.Theorie) vom Schmelzpunkt 195 °C erhalten.

### Beispiel 6: 17β-Acetoxy-1α-methyl-5α-androstan-3-on

Variante 1 (in Dioxan): 3.30 g (10 mmol) 17β-Acetoxy-androst-1-en-3-on werden in 20 ml getrocknetem Dioxan gelöst und mit 143.3 mg (1 mmol) Kupfer-I-bromid versetzt. Unter Stickstoffatmosphäre werden 9.4 ml einer 10%igen Lösung Trimethylaluminium in Toluol über 10 Minuten zu der Reaktion gegeben, daß die Temperatur nicht über 30°C ansteigt. Anschließend wird noch 2 Stunden bei 30°C nachgerührt. Zur Hydrolyse werden 0.54 ml Wasser gelöst in 5 ml Dioxan zur Reaktion gegeben und 10 Minuten nachgerührt. Der Feststoff wird über Kieselgur abgesaugt und mit 20 ml Dioxan nachgewaschen. Das Filtrat wird eingeengt und das Rohprodukt (3.5 g) an Kieselgel mit Hexan/Ethylacetatgemischen als Eluens chromatographiert werden. Nach Einengen der Fraktionen und Umkristallisation aus Aceton werden 3.1 g 17β-Acetoxy-1α-methyl-androstan-3-on (89 % der Theorie) vom Schmelzpunkt 180°C erhalten.

Variante 2 (in Ethylacetat): 66.1 g (0.2 mol) 17β-Acetoxy-androst-1-en-3-on werden in 350 ml trockenem Ethylacetat gelöst und mit 1.43 g (10 mmol) Kupfer-I-bromid versetzt. Unter Stickstoffatmosphäre werden 188 ml (0.22 mol) einer 10%igen Lösung Trimethylaluminium in Toluol über 15 Minuten zu der Reaktion gegeben, daß die Temperatur nicht über 25°C ansteigt. Anschließend wird noch 30 Minuten nachgerührt. Zur Hydrolyse werden 15 ml Wasser zur Reaktion gegeben und noch 40 Minuten nachgerührt. Der Feststoff wird abgesaugt und 3 mal mit je 200 ml Dioxan ausgerührt. Nach Einengen der vereinigten organischen Phasen werden 65.6 g 17β-Acetoxy-1α-methyl-androstan-3-on (95% der Theorie) vom Schmelzpunkt 181°C erhalten.

### Beispiel 7: 16α-Methyl-pregna-1,4-dien-20-on

3.1 g (10 mmol) Pregna-1,4,16-trien-20-on werden in 25 ml Dioxan mit 143.4 mg CuBr versetzt. Unter Stickstoffatmosphäre werden 9.4 ml (11 mmol) einer 10%igen Lösung Trimethylaluminium in Toluol zu der Reaktion gegeben und 30 Minuten bei 20 - 30°C gerührt. Zur Hydrolyse werden 0.54 ml Wasser gelöst in 5 ml Dioxan zu der Reaktion gegeben. Es wird 10 Minuten nachgerührt und der Niederschlag abfiltriert. Der Niederschlag wird mit wenig Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 2.6 g 16α-Methyl-pregna-1,4-dien-20-on (80 % der Theorie) vom Schmelzpunkt 175 °C erhalten.

Die Beispiele 1-7 lassen sich analog in anderen Lösungsmittel wie THF, Dimethoxyethan oder Ethylacetat mit CuBr bzw. CuCN sowie anderen Kupfer-Salzen wie z.B. CuBr₂ oder Bis-(salicylaldehydato)-Kupfer oder Bis-(N-isopropyl-salicylidenaminato)-Kupfer, deren Herstellung in J.Chem.Soc., 1964, 276, beschrieben ist, mit Trimethylaluminium als Methylierungsmittel durchführen.

### Beispiel 8: 3-Acetoxy-1α-methyl-androsta-2,4-dien-17-on

14.2 g (50 mmol) Androsta-1,4-dien-3,17-dion werden unter Stickstoffatmosphäre in 100 ml wasserfreiem Dioxan gelöst. 716 mg (5 mmol) Kupfer-I-bromid werden zugegeben und die Lösung auf 25 °C erhitzt. Anschließend werden 47 ml (55 mmol) einer 10% igen Lösung Trimethylaluminium in Toluol zu der Reaktion gegeben, so daß die Temperatur nicht über 35 °C ansteigt. Anschließend wird noch 1.5 h bei 35°C nachgerührt. Bei 35°C werden 16.84 g (0.165 mol) Essig- säureanhydrid zugegeben und die Reaktionslösung 1 h bei 30 °C nachgerührt. Zur Vervollständigung der Reaktion wird anschließend noch 15 min auf 60°C erhitzt. Nach Abkühlen der Lösung auf 20°C werden zur Hydrolyse 4 ml Wasser gelöst in 20 ml Dioxan zur Reaktion gegeben und die Lösung 15 Minuten nachgerührt. Der anorganische Feststoff wird über Kieselgur abgesaugt und der Feststoff mit 50 ml Dioxan nachgewaschen. Nach Einengen der Lösung am Rotationsverdampfer werden 18 g Rohprodukt erhalten, die an Kieselgel mit Hexan und steigendem Anteil Ethylacetat als Eluens chromatographiert werden. Nach Einengen der Fraktionen werden 11.4 g 3-Acetoxy-1α-methyl-androsta-2,4-dien-17-on (70% der Theorie) als viskoses Material erhalten, das aus Diisopropylether umkristallisiert werden kann. ¹H-NMR (CDCl₃): δ=0.89 (d,J= 7Hz, 3H,1-CH₃), 0.91 (s, 3H, 19-CH₃), 1.05 (s, 3H, 18-CH₃), 2.15 (s, 3H, CH₃CO-O-), 1.0-2.6 (m, 16H), 5.2-5.4 (m, 2H, H-2,H-4).

### Beispiel 9: 3-Methylcyclohexanon

4.8 g (50 mmol) Cyclohex-2-en-1-on und 716 mg CuBr werden bei 20°C in 100 ml absolutem Tetrahydrofuran unter Stickstoffatmosphäre gelöst. Bei 20°C werden 47 ml (55 mmol) einer 10% igen Lösung Trimethylaluminium in Toluol zu der Reaktion gegeben, so daß die Temperatur nicht über 20 °C ansteigt. Nach beendeter Zugabe wird noch 30 min bei 20°C nachgerührt. Zur Hydrolyse werden 4 ml Wasser gelöst in 20 ml Tetrahydrofuran zugegeben und die Lösung 15 min bei Raumtemperatur gerührt. Der anorganische Feststoff wird abfiltriert und mit 50 ml Tetrahydrofuran nachgewaschen. Nach Einengen der Lösung werden 5.5 g Rohprodukt erhalten, die bei 70°C und 2,7 x 10³ Pa (20 Torr) destilliert werden. Es werden 4.0 g 3-Methylcyclohexanon (71 % d. Theorie) erhalten.

### Beispiel 10: 1α-Methyl-androsta-4,6-dien-3,17-dion

2.82 g (10 mmol) Androsta-1,4,6-trien-3,17-dion werden in 20 ml Dioxan bei 20°C gelöst und mit 143.3 mg (1 mmol) Kupfer-I-bromid versetzt. Bei Raumtemperatur werden unter Stickstoffatmosphäre 9.4 ml (11 mmol) einer 10%igen Lösung Trimethylaluminium in Toluol zu der Reaktion gegeben und 10 Minuten bei 20°C gerührt. Zur Hydrolyse werden 1 ml Wasser gelöst in 5 ml Dioxan vorsichtig zu der Reaktion gegeben. Es wird 10 Minuten nachgerührt und der anorganische Niederschlag abfiltriert mit Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 2.97 g 1α-Methyl-androsta-4,6-dien-3,17-dion (99 % der Theorie) vom Schmelzpunkt 171 °C erhalten.

### Beispiel 11: 3-Acetoxy-16α-methyl-pregn-5-en-20-on (16α-Methyl-pregn-5-enolon-3-acetat)

3.56 g (10 mmol) 3-Acetoxy-pregna-5,16-dien-20-on werden in 20 ml Dioxan bei 20°C gelöst und mit 143.3 g (1 mmol) Kupfer-I-bromid versetzt. Bei Raumtemperatur werden unter Stickstoffatmosphäre 9.4 ml (11 mmol) einer 10%igen Lösung Trimethylaluminium in Toluol zu der Reaktion gegeben und nach beendeter Zugabe 10 Minuten bei 20°C gerührt. Zur Hydrolyse der Reaktionslösung wird 1 ml Wasser gelöst in 5 ml Dioxan vorsichtig zu der Reaktion gegeben. Es wird 10 Minuten nachgerührt und der anorganische Niederschlag abfiltriert mit Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 1.12 g 3-Acetoxy-16α-methyl-pregn-5-en-20-on (30 % der Theorie) erhalten.

### Beispiel 12: 1α-Ethylandrost-4-en-3,17-dion

2.84 g (10 mmol) Androsta-1,4-dien-3,17-dion werden unter Stickstoffatmosphäre in 20 ml wasserfreiem Dioxan gelöst. 143 mg (1 mmol) Kupfer-I-bromid werden zugegeben und die Lösung auf 25 °C erwärmt. Anschließend werden 10 ml (10 mmol) einer 1 molaren Lösung Triethylaluminium in Hexan zu der Reaktion gegeben, so daß die Temperatur nicht über 30 °C ansteigt. Anschließend wird noch 1.5 h bei 30 °C nachgerührt. Zur Hydrolyse wird 1 ml Wasser gemischt mit 5 ml Dioxan zur Reaktion gegeben und die Lösung noch 15 min gerührt. Der anorganische Feststoff wird abgesaugt und mit 30 ml Dioxan nachgewaschen. Nach Einengen der Dioxanlösung werden 3 g Rohprodukt erhalten, die an Kieselgel mit einem Hexan/Ethylacetat- Gemisch unter steigendem Anteil Ethylacetat als Eluens chromatographiert werden. Nach Einengen der Fraktionen werden 2.67 g 1α-Ethylandrost-4-en-3,17-dion (85 % der Theorie vom Schmelzpunkt 168°C erhalten.

### Beispiel 13: 1α-Ethyl-androsta-4,6-dien-3,17-dion

2.82 g (10 mmol) Androsta-1,4,6-trien-3,17-dion werden in 20 ml Dioxan bei 20°C gelöst und mit 143.3 mg (1 mmol) Kupfer-I-bromid versetzt. Bei Raumtemperatur werden unter Stickstoffatmosphäre 11 ml (11 mmol) einer 1 molaren Lösung Triethylaluminium in Hexan zu der Reaktion gegeben und 2 Stunden bei 30°C gerührt. Zur Hydrolyse werden 1 ml Wasser gelöst in 5 ml Dioxan vorsichtig zu der Reaktion gegeben. Es wird 10 Minuten nachgerührt und der anorganische Niederschlag abfiltriert mit Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 1.93 g 1α-Ethyl-androsta-4,6-dien-3,17-dion (62 % der Theorie) vom Schmelzpunkt 137.1°C erhalten.

### Beispiel 14: 3-Acetoxy-16α-ethyl-pregn-5-en-20-on (16α-Ethyl-pregn-5-enolon-3-acetat)

3.56 g (10 mmol) 3-Acetoxy-pregna-5,16-dien-20-on werden in 20 ml Dioxan bei 20°C gelöst und mit 143.3 g (1 mmol) Kupfer-I-bromid versetzt. Bei Raumtemperatur werden unter Stickstoffatmosphäre 11 ml (11 mmol) einer 1 molaren Lösung Triethylaluminium in Hexan zu der Reaktion gegeben und nach beendeter Zugabe noch 3 Stunden bei 22°C gerührt. Zur Hydrolyse der Reaktionslösung wird 1 ml Wasser gelöst in 5 ml Dioxan vorsichtig zu der Reaktion gegeben. Es wird 10 Minuten nachgerührt und der anorganische Niederschlag abfiltriert und mit Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 1.72 g 3-Acetoxy-16α-ethyl-pregn-5-en-20-on (45 % der Theorie) vom Schmelzpunkt 114.9 °C erhalten.

### Beispiel 15: 4-Phenyl-pentan-2-on

Zu 1.46 g (10 mmol) cis/trans-Benzylidenaceton (3-Phenyl-but-3-en-2-on) in 20 ml abs. Dioxan werden 143 mg CuBr gegeben. Bei Raumtemperatur werden unter Stickstoffatmosphäre 9.4 ml (11 mmol) einer 10%igen Lösung Trimethylaluminium in Toluol zu der Reaktion gegeben und nach beendeter Zugabe noch 10 Minuten bei 22°C gerührt. Zur Hydrolyse der Reaktionslösung wird 1 ml Wasser gelöst in 5 ml Dioxan vorsichtig zu der Reaktion gegeben. Es wird 10 Minuten nachgerührt und der anorganische Niederschlag abfiltriert mit Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 1.21 g 4-Phenyl-pentan-2-on (75 % der Theorie) als farblose Flüssigkeit erhalten.

### Beispiel 16: 4-Phenyl-hexan-2-on

Zu 1.46 g (10 mmol) cis/trans-Benzylidenaceton (3-Phenyl-but-3-en-2-on) in 20 ml abs. Dioxan werden 143 mg CuBr gegeben. Bei Raumtemperatur werden unter Stickstoffatmosphäre 11 ml (11 mmol) einer 1 molaren Lösung Triethylaluminium in Hexan zu der Reaktion gegeben und nach beendeter Zugabe noch 10 Minuten bei 22°C gerührt. Zur Hydrolyse der Reaktionslösung wird 1 ml Wasser gelöst in 5 ml Dioxan vorsichtig zu der Reaktion gegeben. Es wird 10 Minuten nachgerührt und der anorganische Niederschlag abfiltriert mit Dioxan nachgewaschen. Die Dioxanlösung wird am Rotationsverdampfer eingeengt und der Rückstand an Kieselgel mit Ethylacetat/Hexan als Eluens chromatographiert. Nach Einengen der Fraktionen werden 1.37 g 4-Phenyl-hexan-2-on (78 % der Theorie) als farblose Flüssigkeit erhalten.

### Beispiel 17: 1α-Methylandrost-4-en-3,17-dion

Addition an Androsta-1,4-dien-3,17-dion mit CuBr₂ unter Zusatz von Nickel-bis-acetylacetonat [Ni(AcAc)₂]: nur mit [Ni(AcAc)₂] ist eine 1,4-Addition in Aust.J.Chem.,1975, 28, 817 beschrieben. Die dort beschriebene Reaktionen beziehen sich auf 3-Keto-△⁴-steroide, die dort erzielten Ausbeuten betragen 30-40% d.Th.. Durch CuBr₂-Zusatz verläuft die Reaktion mit höherer Ausbeute und verbesserter Selektivität als unter Ni(AcAc)₂-Katalyse.

2.84 g (10 mmol) Androsta-1,4-dien-3,17-dion werden unter Stickstoffatmosphäre in 10 ml wasserfreiem Tetrahydrofuran gelöst. 223 mg (1 mmol) Kupfer-II-bromid (CuBr₂) und 28.4 mg Ni(AcAc)₂ werden zugegeben und die Lösung auf -20°C abgekühlt. Bei -20°C werden 8.5 ml (10 mmol) einer 10% igen Lösung Trimethylaluminium in Toluol zu der Reaktion gegeben und noch 30 Minuten unter Erwärmen auf 0°C nachgerührt. Zur Hydrolyse wird 1 ml Wasser gelöst in 2 ml Tetrahydrofuran zur Reaktion gegeben und die Lösung noch 15 min nachgerührt. Der anorganische Feststoff wird abgesaugt und mit 30 ml Dioxan nachgewaschen. Nach Einengen der Lösung werden 3.2 g Rohprodukt erhalten, die an Kieselgel mit Hexan/Ethylacetat- Gemischen als Eluens chromatographiert werden. Nach Einengen der Fraktionen werden 2.2 g 1α-Methylandrost-4- en-3,17-dion (74 % der Theorie) vom Schmelzpunkt 154 °C sowie 0.55 g 5β-Methylandrost-1-en-3,17-dion (18.5 % der Theorie) erhalten.

### Versuche zur Umsetzung von α,β-ungesättigten Ketonen mit Kupfer-II-bis-acetylacetonat und Trimethylaluminium

### Beispiel 18: Versuch zur Bildung von 1α-Methylandrost-4-en-3,17-dion

2.84 g (10 mmol) Androsta-1,4-dien-3,17-dion werden unter Stickstoffatmosphäre in 10 ml wasserfreiem Dioxan gelöst. 260 mg (1 mmol) Kupfer-II-bis-acetylacetonat werden zugegeben.

Anschließend werden 47 ml (55 mmol) einer 10%igen Lösung Trimethylaluminium in Toluol zu der Reaktion gegeben, so daß die Temperatur nicht über 35 °C ansteigt. Anschließend wird noch 6 h bei 35°C gerührt. Stündlich wurde die Reaktion mit DC (Kieselgel-HPTLC, Ethylacetat/Hexan 1:1 als Eluens) verfolgt. Es war keine Bildung von 1α-Methylandrost-4-en-3,17-dion festzustellen, lediglich war neben unumgesetztem Ausgangsmaterial zu 30% 3-Exomethylen-androsta-1,4-dien-17-on entstanden.

### Beispiel 19: Versuch Umsetzung von 3,5,5-Trimethylcyclohex-2-en-1-on (Isophoron) zu 3,3,5,5-Tetrametylcyclohexan-1-on mit Kupfer-II-bis-acetylacetonat und Trimethylaluminium nach E.C. Ashby et al.; J.Org.Chem., 1974, 39, 3297.

1.38 g 3,5,5-Trimethylcyclohex-2-en-1-on (Isophoron) werden bei in 10 ml Ether gelöst mit 0.3 mmol Cu(AcAc)₂ versetzt. Bei 22°C werden 9.35 ml einer 10%igen Lösung Trimethylaluminium in Toluol zugegeben. Nach 2 h bei 22°C erfolgt Kontrolle der Reaktion auf Umsetzung. Die Reaktion wird hydrolysiert, mit Ether extrahiert und das Produkt mit NMR untersucht. Es wurde lediglich Ausgangsmaterial (3,5,5-Trimethylcyclohex-2-en-1-on) zurückgewonnen, eine Umsetzung mit Kupfer-II-bis-acetylacetonat als Katalysator war wie die Beispiele 18 und 19 zeigten, nicht durchführbar.

### Beispiel 20: Darstellung von 1-Methyl-androsta-1,2-dien-3,17-dion (Atamestan) gemäß Deutscher Patentanmeldung P 4015247.2

### a) 2β-Jod-1α-methylandrost-4-en-3,17-dion

17.1 g (50 mmol) 2-Acetoxy-1α-methyl-androsta-2,4-dien-17-on (Beispiel 8) werden in 85 ml Aceton gelöst und mit 4.92 g (60 mmol) wasserfreiem Natriumacetat versetzt. Nach Abkühlen auf -10°C werden unter Stickstoffatmosphäre 8.12 g (55 mmol) Chlorjod zugegeben. Es wird 30 Minuten bei -10°C nachgerührt.

Die Reaktionslösung wird unter Rühren in 0.5 l mit 2 g Natriumthiosulfat versetztem Eiswasser gegeben. Der Feststoff wird abgesaugt und mit Wasser nachgewaschen. Nach Trocknen der Substanz werden 20 g Produkt (94 % der Theorie) vom Schmelzpunkt 119 °C (Zers.) erhalten.

### b) 1-Methylandrosta-1,4-dien-3,17-dion (Atamestan)

Zu einer auf 130°C vorgeheizten Suspension von 0.73 g (10 mmol) Lithiumcarbonat in 10 ml Dimethylformamid werden 2.13 g (5 mmol) 2β-Jod-1α-methylandrost-4-en-3,17-dion aus a) eingetragen und 1.5 Stunden bei dieser Temperatur gerührt. Nach Abkühlen wird die Reaktionslösung auf 30 ml Wasser gegeben, mit Ethylacetat extrahiert und nach Trocknen über Natriumsulfat eingeengt. Das Rohprodukt wird an Kieselgel mit Ethylacetat/Hexan als Laufmittel chromatographiert. Nach Umkristallisation aus Ethylacetat werden 1.1 g 1-Methylandrosta-1,4-dien-3,17-dion (74 % der Theorie) vom Schmelzpunkt 169°C erhalten.

### Beispiel 21: 17β-Acetoxy-2α-brom-1α-methyl-5α-androstan-3-on

Zu 818 mg (2 mmol) 17β-Acetoxy-2-brom-5α-androst-1-en-3-on (hergestellt nach J. Org.Chem.1982, 47, 5090; aus 17β-Acetoxy-2-brom-5α-androst-1-en-3-on) in 4 ml Ethylacetat werden bei Raumtemperatur 14.3 mg (0.1) mmol) CuBr gegeben. Bei Raumtemperatur werden 1.9 ml (2.2 mmol) einer 10%igen Lösung Trimethylaluminium in Toluol zugetropft. Es wird 45 min bei Raumtemperatur gerührt. Zur Hydrolyse werden 0.18 ml Wasser zugegeben, der Feststoff wird abgesaugt und mit Ethylacetat nachgewaschen. Die Lösung wird eingeengt und der Rückstand an Silicagel mit Hexan/Ethylacetat als Eluens chromatographiert. Nach Einengen der Chromatographiefraktionen werden 600 mg 17β-Acetoxy-2α-brom-1α-methyl-5α-androstan-3-on erhalten.

### Beispiel 22: 17-Acetoxy-1α-ethyl-5α-androstan-3-on

Zu 3.3 g (10 mmol) 17-Acetoxy-5α-androst-1-en-3-on in 15 ml Ethylacetat und 44.78 mg (0.5 mmol) Kupfercyanid (CuCN) werden bei Raumtemperatur 5.78 ml (11 mmol) einer 1.9 molaren Lösung Triethylaluminium in Toluol gegeben. Es wird noch 12 h bei 25°C gerührt. Die Reaktionslösung wird mit 1 ml Wasser hydrolysiert und 15 Minuten nachgerührt. Der anorganische Feststoff abfiltriert. Der Feststoff wird mit Ethylacetat nachgewaschen. Nach Abdampfen des Lösungsmittels und Chromatographie der Substanz an Silicagel mit Hexan/ Ethylacetat als Eluens werden 1.44 g (39% der Theorie) 17-Acetoxy-1α-ethyl-5α-androstan-3-on erhalten.

### Beispiel 23: 1-Methyl-7,7-(2,2-dimethyltrimethylen-dioxy)-cis-bicyclo [3.3.0] octan-3-on

Zu 444.6 mg (2 mmol) 7,7-(2,2-Dimethyltrimethylen-dioxy)-cis-bicyclo [3.3.0] oct-1-en-3-on und 28.7 mg (0.2 mmol) CuBr in 3 ml Dioxan werden 1.9 ml (2.2 mmol) Trimethylaluminium als 10%ige Lösung in Toluol gegeben und die Lösung bei Raumtemperatur gerührt. Nach 15 Minuten wird mit Wasser hydrolysiert, das Produkt mit Ethylacetat extrahiert und an Silicagel mit Hexan unter steigendem Anteil Ethylacetat als Eluens chromatographiert. Nach Einengen der Fraktionen werden 290 mg (61% der Theorie) 1-Methyl-7,7-(2,2-dimethyltrimethylen-dioxy)-cis-bicyclo[3.3.0] octan-3-on erhalten.

### Beispiel 24: 1-Ethyl-7,7-(2,2-dimethyltrimethylen-dioxy)-cis-bicyclo[3.3.0]octan-3-on

Zu 444.57 mg (2 mmol) 7,7-(2,2-Dimethyltrimethylen-dioxy)-cis-bicyclo[3.3.0]oct-1-en-3-on und 17.7 mg (0.1 mmol) CuBr in 4 ml abs. Ethylacetat werden 1.15 ml (2.2 mmol) einer 1.9 molaren Lösung Triethylaluminium in Toluol gegeben. Es wird noch 2 h bei 25°C gerührt. Die Reaktionslösung wird dann mit 20 ml Wasser hydrolysiert. Es wird noch 15 Minuten nachgerührt und das Produkt mit Ethylacetat extrahiert. Nach Abdampfen des Lösungsmittels und Chromatographie der Substanz an Silicagel mit Hexan/Ethylacetat als Eluens werden 0.3 g 1-Ethyl-7,7-(2,2-dimethyltrimethylendioxy)-cis-bicyclo[3.3.0]octan-3-on (60% der Theorie) erhalten.

### Beispiel 25: 2-tert-Butyl-5-methyl-cyclohexanon

Zu 4.56 g (30 mmol) 2-Isopropyliden-5-methyl-cyclohexan-1-on (Pulegon) und 214.5 mg (1.5 mmol) CuBr in 30 ml Ethylacetat werden 28.5 ml (33 mmol) Trimethylaluminium als 10%ige Lösung in Toluol getropft. Die Reaktionslösung wird noch 1h bei 25 °C gerührt. Zur Hydrolyse werden vorsichtig 2 ml Wasser zugegeben und noch 15 min nachgerührt. Der anorganische Feststoff wird abgesaugt, mit Ethylacetat nachgewaschen und die Lösung im Vakuum eingeengt. Destillation des Rohproduktes bei 120°C/6 Torr ergibt 3.4 g 2-tert-Butyl-5-methyl-cyclohexanon (70% der Theorie) als Isomerengemisch.

### Beispiel 26: 3,3,5,5-Tetramethylcyclohexanon

1.38 g 3,5,5-Trimethylcyclohex-2-en-1-on (Isophoron) werden in 10 ml Dioxan gelöst und 143 mg (1 mmol) CuBr zugegeben. Bei Raumtemperatur werden 9.4 ml (11 mmol) Trimethylaluminium 10%ig in Toluol zugegeben. Die Lösung wird 1 h bei Raumtemperatur gerührt. Zur Hydrolyse wird 1 ml Wasser zugegeben, die Lösung wird mit Ethylacetat verdünnt, der Feststoff abfiltriert und mit Ethylacetat nachgewaschen. Nach Abdampfen des Lösungsmittels und Chromatographie der Substanz an Silicagel mit Hexan/Ethylacetat als Eluens werden 1.20 g (78% der Theorie) 3,3,5,5-Tetramethylcyclohexanon erhalten.

### Beispiel 27: 17β-Acetoxy-1α-methyl-5α-androstan-3-on

### a) in situ Herstellung von Dimethylaluminium-ethoxid

Zu 11 mmol (9,5 ml) einer 10%igen Lösung von Trimethylaluminium in Toluol werden bei 0° C 0.506 g (11 mmol) abs. Ethanol gegeben. Es wird noch 10 Minuten nachgerührt.

### b) Umsetzung zu 17β-Acetoxy-1α-methyl-5α-androstan-3-on

Zu der Lösung a) werden 3.3 g (10 mmol) 17β-Acetoxy-5a-androst-1-en-3-on in 15 ml Ethylacetat und 143 mg (0.1 mmol) CuBr gegeben. Es wird noch 16 h bei 25°C gerührt. Die Reaktionslösung wird mit 1 ml Wasser hydrolysiert, 15 Minuten nachgerührt und der anorganische Feststoff abfiltriert. Der Feststoff wird mit Ethylacetat nachgewaschen. Nach Abdampfen des Lösungsmittels werden 3.5 g Substanz erhalten, die aus Aceton umkristallisiert werden. Nach Absaugen der Kristalle werden 2.9 g 17-Acetoxy-1α-methyl-5α-androstan-3-on vom Schmelzpunkt 179-180°C erhalten.

## Patentansprüche

1. Methylierungs- bzw. Ethylierungsmittel, enthaltend Trimethylaluminium oder Dimethylzink bzw. Triethylaluminium als Methyl- bzw. Ethylquelle, dadurch gekennzeichnet, daß es zusätzlich katalytische Mengen einer oder mehrerer Kupfer-I- und/oder Kupfer-II-Verbindungen enthält.

2. Methylierungs- bzw. Ethylierungsmittel, enthaltend ein Aluminium-Reagenz Alk₃₋ₙ AlOEtₙ, worin Alk eine Methyl- oder Ethylgruppe und OEt eine Ethoxygruppe bedeuten und n gleich 1 oder 2 ist, als Methyl- oder Ethylquelle, dadurch gekennzeichnet, daß es zusätzlich katalytische Mengen einer oder mehrerer Kupfer-I- und/oder Kupfer-II-Verbindungen enthält.

3. Alkylierungsmittel nach Anspruch 1 oder 2, dadurch gekennzeichnet, daß es insgesamt 1-10 Mol-% der Kupfer-I und/oder Kupfer-II-Verbindung bezogen auf die zu alkylierende Verbindung enthält.

4. Alkylierungsmittel nach Anspruch 1, 2 oder 3, dadurch gekennzeichnet, daß es als Kupfer-I und/oder Kupfer-II-Verbindung eine oder mehrere Verbindung(en) der allge meinen Formel I
CuX oder CuX₂ (I)
worin X einen einwertigen Rest darstellt und für Chlor, Brom, Iod, Cyano, den Thienyl- Phenyl-, einen Alkoxy-, Thioalkoxy-, wobei der darin enthaltene Alkylrest 1 bis 8 Kohlenstoffatome besitzt und gegebenenfalls verzweigt und/oder ungesättigt ist, für einen substituierten Alkinylrest R-C≡C-, wobei R einen Phenyl- oder einen gegebenenfalls verzweigten C₁-C₈-Alkylrest bedeutet, oder den Rest einer anorganischen Säure oder einer Carbonsäure oder für einen zweizähnigen, über Sauerstoff- und/oder Stickstoffatome koordinierenden Komplexliganden, ausgenommen den Liganden Acetylacetonat im Fall des zweiwertigen Kupfers steht und/oder eine oder mehrere Verbindung(en) der allgemeinen Formel II
Cu₂Y oder CuY (II)
worin Y einen zweiwertigen Rest darstellt und für Sauerstoff oder Schwefel steht, enthält.

5. Alkylierungsmittel nach Anspruch 4, dadurch gekennzeichnet, daß es Kupfer-I- und/oder Kupfer-II-chlorid und/oder -bromid und/oder Kupfer-I-cyanid enthält.

6. Alkylierungsmittel nach einem der vorstehenden Ansprüche 1 bis 5, dadurch gekennzeichnet, daß es zusätzlich bis 1 Mol% an Nickelsalz(en) enthält.

7. Alkylierungsmittel nach Anspruch 6, dadurch gekennzeichnet, daß es als Nickelsalz Nickel-II-acetylacetonat, Nickel-bis-triphenylphosphan-dichlorid oder Nickeldichlorid. enthält.

8. Verfahren zur 1,4-Addition einer Methyl- oder Ethylgruppe an ein α,β-ungesättigtes oder ein α,β-doppelt ungesättigtes Keton oder an einen α,β-ungesättigten Aldehyd, dadurch gekennzeichnet, daß das α,β-ungesättigte Keton, das α,β-doppelt ungesättigte Keton oder der α,β-ungesättigte Aldehyd mit Aluminiumtrimethyl oder Zinkdimethyl bzw. Aluminiumtriethyl in Gegenwart einer katalytischen Menge einer oder mehrerer Kupfer-I- und/oder Kupfer-II-Verbindungen alkyliert wird.

9. Verfahren zur 1,4-Addition einer Methyl- oder Ethylgruppe an ein α,β-ungesättigtes oder ein α,β-doppelt ungesättigtes Keton oder an einen α,β-ungesättigten Aldehyd, dadurch gekennzeichnet, daß das α,β-ungesättigte Keton, das α,β-doppelt ungesättigte Keton oder der α,β-ungesättigte Aldehyd mit einem Aluminium-Reagenz Alk₃₋ₙ AlOEtₙ, worin Alk eine Methyl- oder Ethylgruppe und OEt eine Ethoxygruppe bedeuten und n gleich 1 oder 2 ist, in Gegenwart einer katalytischen Menge einer oder mehrerer Kupfer-I- und/oder Kupfer-II-Verbindungen alkyliert wird.

10. Verfahren nach Anspruch 8 oder 9, dadurch gekennzeichnet, daß in Gegenwart von insgesamt 1-10 Mol% der Kupfer-I- und/oder Kupfer-II-Verbindung bezogen auf das zu alkylierende α,β-ungesättigte Keton alkyliert wird.

11. Verfahren nach Anspruch 8, 9 oder 10, dadurch gekennzeichnet, daß in Gegenwart einer oder mehrerer Verbindung(en) der allgemeinen Formel I
CuX oder CuX₂ (I)
worin X einen einwertigen Rest darstellt und für Chlor, Brom, Iod, Cyano, den Thienyl-, Phenyl-, einen Alkoxy-, Thioalkoxy, wobei der Alkylrest 1 bis 8 Kohlenstoff atome besitzt und gegebenenfalls verzweigt und/oder ungesättigt ist, einen substituierten Alkinylrest R-C≡C-, wobei R einen Phenyl- oder einen gegebenenfalls verzweigten C₁-C₈-Alkylrest bedeutet, oder den Rest einer anorganischen Säure oder einer Carbonsäure oder für einen zweizähnigen, über Sauerstoff- und/oder Stickstoffatome koordinierenden Komplexliganden, ausgenommen den Liganden Acetylacetonat im Fall des zweiwertigen Kupfers, steht, und/oder einer oder mehrerer Verbindung(en) der allgemeinen Formel II
Cu₂Y oder CuY (II)
worin Y einen zweiwertigen Rest darstellt und für Sauerstoff oder Schwefel steht, alkyliert wird.

12. Verfahren nach Anspruch 11, dadurch gekennzeichnet, daß in Gegenwart von Kupfer-I- und/oder Kupfer-II-chlorid und/oder -bromid und/oder Kupfer-I-cyanid alkyliert wird.

13. Verfahren nach einem der vorstehenden Ansprüche 8 bis 12, dadurch gekennzeichnet, daß zusätzlich bis 1 Mol% Nickelsalz zugegen ist.

14. Verfahren nach Anspruch 13, dadurch gekennzeichnet, daß als Nickelsalz Nickel-II-acetylacetonat, Nickel-bis-triphenylphosphan-dichlorid oder Nickeldichlorid zugegen ist.

15. Verfahren nach einem der vorstehenden Ansprüche 8 bis 14, dadurch gekennzeichnet, daß es in Tetrahydrofuran, Dioxan, Dimethoxyethan oder Toluol als Lösungsmittel durchgeführt wird.

16. Verfahren nach einem der vorstehenden Ansprüche 8 bis 14, dadurch gekennzeichnet, daß es in Ethylacetat als Lösungsmittel durchgeführt wird.

17. Verfahren nach einem der vorstehenden Ansprüche 8 bis 16, dadurch gekennzeichnet, daß es bei einer Reaktionstemperatur von 0° C bis 50° C durchgeführt wird.

18. Verfahren nach einem der vorstehenden Ansprüche 8 bis 17, dadurch gekennzeichnet, daß das α,β-ungesättigte Keton ein 3-Keto-1,4-dien-Steroid, ein 3-Keto-1-en-Steroid, ein 3-Keto-4-en-Steroid oder ein 17-Acyl-16-en-Steroid ist.

19. Verfahren zur Herstellung von 1α-Methylandrost-4-en-3,17-dion als Zwischenprodukt zur Herstellung von 1α-Methyl-17β-hydroxy-5α-androstan-3-on (Mesterolon), dadurch gekennzeichnet, daß Androsta-1,4-dien-3,17-dion gemäß einem der Anprüche 8-17 methyliert wird.

20. Verfahren zur Herstellung eines 3-Acyloxy-1α-methyl-androsta-2,4-dien-17-ons als Zwischenprodukt für die Herstellung von 1-Methyl-androsta-1,4-dien-3,17-dion (Atamestan), dadurch gekennzeichnet, daß Androsta-1,4-dien-3,17-dion gemäß einem der Ansprüche 8-17 methyliert und das nach der Methylierung in der Reaktionsmi schung vorliegende Enolat mit einem Carbonsäureanhydrid- oder chlorid einer gerad- oder verzweigtkettigen Alkancarbonsäure mit 2 bis 8 Kohlenstoffatomen oder der Benzoesäure, abgefangen wird.

21. Verfahren nach Anspruch 20, dadurch gekennzeichnet, daß das Enolat mit Acetanhydrid oder Acetylchlorid abgefangen wird.

## Claims

1. Methylating or ethylating agent, comprising as the source of methyl or ethyl trimethylaluminium or dimethylzinc, or triethylaluminium, respectively, characterised in that it comprises additionally catalytic amounts of one or more copper(I) and/or copper(II) compound(s).

2. Methylating or ethylating agent, comprising as the source of methyl or ethyl an aluminium reagent Alk₃₋ₙAlOEtₙ wherein Alk is a methyl or ethyl group, OEt is an ethoxy group and n is 1 or 2, characterised in that it comprises additionally catalytic amounts of one or more copper(I) and/or copper(II) compound(s).

3. Alkylating agent according to claim 1 or claim 2, characterised in that it comprises a total of from 1 to 10 mol % of the copper(I) and/or copper(II) compound, based on the compound that is to be alkylated.

4. Alkylating agent according to claim 1, 2 or 3, characterised in that it comprises as copper(I) and/or copper(II) compound one or more compound(s) of the general formula I
CuX or CuX₂ (I)
wherein X represents a monovalent radical and is chlorine, bromine, iodine, cyano, a thienyl radical, a phenyl radical, an alkoxy or thioalkoxy radical wherein the alkyl radical has from 1 to 8 carbon atoms and is optionally branched-chained and/or unsaturated, a substituted alkynyl radical R-C≡C- wherein R is a phenyl radical or an optionally branched-chained C₁C₈alkyl radical, or the radical of an inorganic or carboxylic acid, or, with the exception of the acetylacetonate ligand in the case of the bivalent copper, X is a bidentate complex ligand that co-ordinates by way of oxygen and/or nitrogen atoms,
and/or one or more compound(s) of the general formula II
Cu₂Y or CuY (II)
wherein Y represents a bivalent radical and is oxygen or sulphur.

5. Alkylating agent according to claim 4, characterised in that it comprises copper(I) and/or copper(II) chloride and/or bromide and/or copper(I) cyanide.

6. Alkylating agent according to any one of the preceding claims 1 to 5, characterised in that it comprises additionally up to 1 mol % of nickel salt(s).

7. Alkylating agent according to claim 6, characterised in that it comprises as nickel salt nickel(II) acetylacetonate, nickel bistriphenylphosphanedichloride or nickel dichloride.

8. Process for the 1,4-addition of a methyl or ethyl group to an α,β-unsaturated or α,β-doubly-unsaturated ketone or to an α,β-unsaturated aldehyde, characterised in that the α,β-unsaturated ketone, the α,β-doubly-unsaturated ketone or the α,β-unsaturated aldehyde is alkylated with aluminium trimethyl or zinc dimethyl or with aluminium triethyl in the presence of a catalytic amount of one or more copper(I) and/or copper(II) compound(s).

9. Process for the 1,4-addition of a methyl or ethyl group to an α,β-unsaturated or α,β-doubly-unsaturated ketone or to an α,β-unsaturated aldehyde, characterised in that the α,β-unsaturated ketone, the α,β-doubly-unsaturated ketone or the α,β-unsaturated aldehyde is alkylated with an aluminium reagent Alk₃₋ₙAlOEtₙ wherein Alk is a methyl or ethyl group, OEt is an ethoxy group and n is 1 or 2, in the presence of a catalytic amount of one or more copper(I) and/or copper(II) compound(s).

10. Process according to claim 8 or claim 9, characterised in that alkylation is carried out in the presence of a total of from 1 to 10 mol % of the copper(I) and/or copper(II) compound, based on the α,β-unsaturated ketone that is to be alkylated.

11. Process according to claim 8, 9 or 10, characterised in that alkylation is carried out in the presence of one or more compound(s) of the general formula I
CuX or CuX₂ (I)
wherein X represents a monovalent radical and is chlorine, bromine, iodine, cyano, a thienyl radical, a phenyl radical, an alkoxy or thioalkoxy radical wherein the alkyl radical has from 1 to 8 carbon atoms and is optionally branched-chained and/or unsaturated, a substituted alkynyl radical R-C≡C- wherein R is a phenyl radical or an optionally branched-chained C₁-C₈alkyl radical, or the radical of an inorganic or carboxylic acid, or, with the exception of the acetylacetonate ligand in the case of the bivalent copper, X is a bidentate complex ligand that co-ordinates by way of oxygen and/or nitrogen atoms,
and/or one or more compound(s) of the general formula II
Cu₂Y or CuY (II)
wherein Y represents a bivalent radical and is oxygen or sulphur.

12. Process according to claim 11, characterised in that alkylation is carried out in the presence of copper(I) and/or copper(II) chloride and/or bromide and/or of copper(I) cyanide.

13. Process according to any one of the preceding claims 8 to 12. characterised in that additionally up to 1 mol % of nickel salt is present.

14. Process according to claim 13, characterised in that there is present as nickel salt nickel(II) acetylacetonate, nickel bistriphenylphosphanedichloride or nickel dichloride.

15. Process according to any one of the preceding claims 8 to 14, characterised in that it is carried out in tetrahydrofuran, dioxane, dimethoxyethane or toluene as solvent.

16. Process according to any one of the preceding claims 8 to 14, characterised in that it is carried out in ethyl acetate as solvent.

17. Process according to any one of the preceding claims 8 to 16, characterised in that it is carried out at a reaction temperature of from 0°C to 50°C.

18. Process according to any one of the preceding claims 8 to 17, characterised in that the α,β-unsaturated ketone is a 3-keto-1,4-diene steroid, a 3-keto-1-ene steroid, a 3-keto-4-ene steroid or a 17-acyl-16-ene steroid.

19. Process for the preparation of 1α-methylandrost-4-ene-3,17-dione as intermediate in the preparation of 1α-methyl-17β-hydroxy-5α-androstan-3-one (mesterolone), characterised in that androsta-1,4-diene-3,17-dione is methylated according to any one of claims 8 to 17.

20. Process for the preparation of a 3-acyloxy-1α-methyl-androsta-2,4-dien-17-one as intermediate in the preparation of 1-methyl-androsta-1,4-diene-3,17-dione (atamestane), characterised in that androsta-1,4-diene-3,17-dione is methylated according to any one of claims 8 to 17 and the enolate that is present in the reaction mixture after methylation is captured by a carboxylic acid anhydride or chloride of a straight- or branched-chained alkanecarboxylic acid having from 2 to 8 carbon atoms or of benzoic acid.

21. Process according to claim 20, characterised in that the enolate is captured by acetic anhydride or acetyl chloride.

## Revendications

1. Agent de méthylation ou d'éthylation comprenant du triméthylaluminium ou du diméthylzinc, respectivement du triméthylaluminium comme source de méthyle ou d'éthyle, caractérisé en ce qu'il comprend en plus des quantités catalytiques d'un ou plusieurs composés de cuivre-I et/ou de cuivre-II.

2. Agent de méthylation ou d'éthylation comprenant un réactif à base d'aluminium Alc₃₋ₙAlOEtₙ, dans laquelle Alc représente un groupe méthyle ou éthyle et OEt un groupe éthoxy et n vaut 1 ou 2, comme source de méthyle ou d'éthyle, caractérisé en ce qu'il comprend en plus des quantités catalytiques *d'un* ou plusieurs composés de cuivre-I et/ou de cuivre-II.

3. Agent alkylant selon la revendication 1 ou 2, caractérisé en ce qu'il comprend de 1 à 10 % en moles d'un ou plusieurs composés de cuivre-I et/ou de cuivre-II.

4. Agent alkylant selon la revendication 1, 2 ou 3, caractérisé en ce qu'il comprend un ou plusieurs composés de cuivre-I et/ou de cuivre-II de formule générale I
CuX ou CuX₂ (I)
dans laquelle X est un radical monovalent et représente les groupes chloro, bromo, iodo, cyano, les radicaux thiènyle, phényle, alcoxy, thioalcoxy, où le radical alkyle comporte de 1 à 8 atomes carbone et est éventuellement ramifié et/ou insaturé, représente aussi un radical alcynyle R-C≡C- substitué, R étant un phényle ou un radical alkyle C₁-C₈ éventuellement ramifié, ou représente le radical d'un acide minéral ou d'un acide carboxylique, ou est un ligand bidenté de complexe de coordination par les atomes d'oxygène et/ou d'azote, à l'exception du ligand acétylacétonate dans le cas du cuivre bivalent, et/ou un ou plusieurs composés de formule générale II
Cu₂Y ou CuY (II)
dans laquelle Y représente un radical bivalent et est l'oxygène ou le soufre.

5. Agent alkylant selon la revendication 4, caractérisé en ce qu'il contient le chlorure et/ou le bromure de cuivre-I et/ou de cuivre-II et/ou le cyanure de cuivre-I.

6. Agent alkylant selon l'une quelconque des revendications 1 à 5, caractérisé en ce qu'il comprend en plus 1 % en moles de sel de nickel.

7. Agent alkylant selon la revendication 6, caractérisé en ce qu'il comprend comme sel de nickel l'acétylacétonate de nickel-II, le bis-triphenylphosphanedichlorure de nickel ou le chlorure de nickel.

8. Procédé pour l'addition en 1,4 d'un groupe méthyle ou éthyle sur une cétone insaturée en α,β ou sur une cétone à double insaturation en α,β ou sur un aldéhyde insaturé en α,β, caractérisé en que la cétone insaturé en α,β, la cétone à double insaturation en α,β ou l'aldéhyde insaturé en α,β sont alkylés par le triméthylaluminium ou le diméthylzinc en présence d'une quantité catalytique d'un ou plusieurs composés de cuivre-I et/ou de cuivre-II.

9. Procédé pour l'addition en 1,4 d'un groupe méthyle ou éthyle sur une cétone insaturée en α,β ou sur une cétone à double insaturation en α,β ou sur un aldéhyde insaturé en α,β, caractérisé en que la cétone insaturé en α,β, la cétone à double insaturation en α,β ou l'aldéhyde insaturé en α,β sont alkylés par un réactif à base d'aluminium Alc₃₋ₙAlOEtₙ, où Alc est un groupe méthyle ou éthyle et OEt un groupe éthoxy et n vaut 1 ou 2, en présence d'une quantité catalytique d'un ou plusieurs composés de cuivre-I et/ou de cuivre-II.

10. Procédé selon la revendication 8 ou 9, caractérisé en ce qu'on met en oeuvre l'alkylation en présence d'un total de 1 à 10 % en moles du composé de cuivre-I et/ou de cuivre-II par rapport à la cétone insaturé en α,β à alkyler.

11. Procédé selon la revendication 8, 9 et 10, caractérisé en ce qu'on met en oeuvre l'alkylation en présence d'un ou plusieurs composés formule générale I
CuX ou CuX₂ (I)
dans laquelle X est un radical monovalent et représente les groupes chloro, bromo, iodo, cyano, les radicaux thiènyle, phényle, alcoxy, thioalcoxy, où le radical alkyle comporte de 1 à 8 atomes carbone et est éventuellement ramifié et/ou insaturé, représente aussi un radical alcynyle R-C≡C- substitué, R étant un phényl ou un radical alkyle en C₁-C₈ éventuellement ramifié, ou représente le radical d'un acide minéral ou d'un acide carboxylique, ou est un ligand bidenté de complexe de coordination par les atomes d'oxygène et/ou d'azote, à l'exception du ligand acétylacétonate dans le cas du cuivre bivalent, et/ou un ou plusieurs composés de formule générale II
Cu₂Y ou CuY (II)
dans laquelle Y représente un radical bivalent et est l'oxygène ou le soufre.

12. Procédé selon la revendication 11, caractérisé en ce qu'on met en oeuvre l'alkylation en présence du chlorure et/ou du bromure de cuivre-I et/ou de cuivre-II et/ou du cyanure de cuivre-I.

13. Procédé selon l'une quelconque des revendications 8 à 12, caractérisé en ce qu'on ajoute en plus 1% en mole de sel de nickel.

14. Procédé selon la revendication 13, caractérisé en ce qu'on ajoute comme sel de nickel l'acétylacétonate de nickel-II, le bis-triphénylphosphane-dichlorure de nickel ou le dichlorure de nickel.

15. Procédé selon l'une quelconque des revendications 8 à 14, caractérisé en ce qu'il est mis en oeuvre dans du tétrahydrofuranne, du dioxanne, du dioxyméthane ou du toluène comme solvant.

16. Procédé selon l'une quelconque des revendications 8 à 14, caractérisé en ce qu'il est mis en oeuvre dans l'acétate d'éthyle comme solvant.

17. Procédé selon l'une quelconque des revendications 8 à 14, caractérisé en ce qu'il est mis en oeuvre à une température de réaction de 0°C à 50°C.

18. Procédé selon l'une quelconque des revendications 8 à 17, caractérisé en la cétone insaturée en α,β est un stéroïde 3-céto-1,4-diénique, un 3-céto-1-ène-stéroïde, un 3-céto-4-ène-stéroïde ou un 17-acyl-16-ène-stéroïde.

19. Procédé pour la préparation de la 1α-méthylandrost-4-ène-3,17-dione comme produit intermédiaire pour la préparation de la 1α-méthyle-17β-hydroxy-5α-androstan-3-one (mestérolon), caractérisé en ce que l'androsta-1,4-diène-3,17-dione est méthylée conformément à l'une quelconque des revendications 8 à 17.

20. Procédé de préparation d'une 3-acyloxy-1α-méthyl-androsta-2,4-diène-3,17-dione comme produit intermédiaire pour la préparation de la 1-méthyl-androsta-1,4-diéne-3,17-dione (atemestan) caractérisé en ce qu'on effectue la méthylation de l'androsta-1,4-diène-3,17-dione conformément à l'une quelconque des revendications 8 à 17 et que l'on récupère l'énolate, présent dans le mélange réactionnel après la méthylation, conjointement avec le chlorure ou l'anhydride d'acide carboxylique d'un acide alcanecarboxylique linéaire ou ramifié avec 2 à 8 atomes de carbone ou de l'acide benzoïque.

21. Procédé selon la revendication 20, caractérisé en ce que l'on récupère l'énolate avec l'anhydride acétique ou le chlorure d'acétyle.
